(19) **Europäisches Patentamt**

**European Patent Office**

**Office européen des brevets**

(11) Veröffentlichungsnummer: **0 205 770 B1**

(12) **EUROPÄISCHE PATENTSCHRIFT**

(45) Veröffentlichungstag der Patentschrift: **09.10.91**

(21) Anmeldenummer: **86103903.0**

(22) Anmeldetag: **21.03.86**

Die Akte enthält technische Angaben, die nach dem Eingang der Anmeldung eingereicht wurden und die nicht in dieser Patentschrift enthalten sind.

(51) Int. Cl.⁵: **C07D 493/04**, //(C07D493/04, 307:00,307:00)

(54) Verfahren zur Herstellung von 2,5-Dimethyl- bzw. 2,5-Diethyl-1,4;3,6-dianhydrosorbitol.

(30) Priorität: **19.06.85 DE 3521809**

(43) Veröffentlichungstag der Anmeldung:
**30.12.86 Patentblatt 86/52**

(45) Bekanntmachung des Hinweises auf die Patenterteilung:
**09.10.91 Patentblatt 91/41**

(84) Benannte Vertragsstaaten:
**AT BE CH DE FR GB IT LI LU NL SE**

(56) Entgegenhaltungen:
**EP-A- 0 092 998**
**GB-A- 988 123**
**US-A- 3 272 845**
**US-A- 4 322 359**

(73) Patentinhaber: **RÜTGERSWERKE AKTIENGE-SELLSCHAFT**
**Mainzer Landstrasse 217**
**W-6000 Frankfurt am Main 1(DE)**

(72) Erfinder: **Maurer, Manfred, Dr.**
**Bissersheimer Strasse 23**
**W-6719 Kirchheim/Weinstrasse(DE)**
Erfinder: **Orth, Winfried, Dr.**
**Am Schachtelgraben 28**
**W-6733 Hassloch/Pfalz(DE)**
Erfinder: **Fickert, Werner, Dr.**
**Stockacher Strasse 14**
**W-6800 Mannheim(DE)**

## Beschreibung

Die Erfindung betrifft ein neues Verfahren zur Herstellung von 2,5-Dimethyl- bzw. 2,5-Diethyl-1,4;3,6-dianhydrosorbitol. Diese Produktgruppe zeigt außergewöhnliche Verträglichkeiten mit Wasser und mit organischen Lösungsmitteln. Insbesondere das 2,5-Dimethyl-1,4;3,6-dianhydrosorbitol , auch bekannt als Dimethylisosorbid, findet eine breite Verwendung als hochwertiges Lösungsmittel in pharmazeutischen und kosmetischen Präparaten.

Aus US-A-4,322,359 ist ein Verfahren zur Herstellung von Dimethylisosorbid bekannt, bei dem 1,4;3,6-Dianhydrosorbitol in einem Gemisch aus Alkalilauge und einem mit Wasser mischbaren hydroxylgruppenfreien Lösungsmittel gelöst und mit Dimethylsulfat methyliert wird. Das Endprodukt wird durch Extraktion abgetrennt und durch Destillation gereinigt, wobei nach der Destillation das Dimethylisosorbid in einer Ausbeute von 82,6 % erhalten wird.

In ähnlicher Weise wird gemäß EP-A-0 092 998 Isosorbid alkyliert, wobei als Lösungsmittel , Aromaten oder polare hydroxylgruppenfreie aliphatische Lösungsmittel, insbesondere Dimethylsulfoxid eingesetzt werden. Es werden Ausbeuten bis zu 83,5 % erhalten.

Eine derartige Ausbeute ist nicht zufriedenstellend, weil es sich bei diesen Verbindungen um hochwertige Produkte handelt.

Es bestand daher die Aufgabe, das an sich einfache Verfahren zur Methylierung bzw. Ethylierung von 1,4;3,6-Dianhydrosorbitol so zu verbessern, daß die Alkylierungsprodukte in höherer Ausbeute und damit wirtschaftlicher erhalten werden.

Die Aufgabe wird gelöst durch ein Verfahren gemäß den Ansprüchen.

Es ist aus US-A-4,322,359 bekannt, daß das bei der Alkylierungsreaktion eingesetzte organische Lösungsmittel für den Verlauf der Reaktion und damit für die Ausbeute ausschlaggebend ist. Hydroxygruppenhaltige Lösungsmittel führen gemäß der Lehre dieser Schrift infolge von Nebenreaktionen, wenn überhaupt, so doch nur zu einer niedrigen Ausbeute an Dimethylisosorbid und sollten daher nicht eingesetzt werden.

Überraschenderweise aber wurde gefunden, daß beim Einsatz von tert. Butanol oder tert. Amylalkohol als Lösungsmittel bei der Alkylierung von 1,4;3,6-Dianhydrosorbitol nicht nur ein störungsfreier Verlauf der Reaktion erfolgt, sondern daß dabei auch die Ausbeute an 2,5-Dimethyl-bzw 2,5-Diethyl-1,4;3,6-dianhydrosorbitol wesentlich erhöht wird.

Als Alkylierungsmittel können übliche Agenzien für die elektrophile Alkylierung eingesetzt werden, wie z.B. Methyljodid, Dimethylsulfat, Diethylsulfat oder p-Toluolsulfonsäuremethyl-oder ethylester.

Zur Alkylierung wird Isosorbid in der etwa 2- bis 4-fachen Menge tert. Butanol oder tert. Amylalkohol gelöst, wobei das Gemisch zweckmäßigerweise auf 40 - 70 °C erhitzt wird. Danach werden gleichzeitig das entsprechende Alkylierungsmittel und eine wäßrige, alkalische Lösung langsam zugegeben, wobei die alkalische Lösung immer so dosiert sein muß, daß das Reaktionsgemisch alkalisch ist. Als alkalische Lösung sind wäßrige Lösungen alkalisch wirkender Stoffe geeignet, wie z.B. Lösungen von Alkali- oder Erdalkalihydroxiden oder Alkalicarbonaten. Bevorzugt werden 40 bis 60%ige Natrium- oder Kaliumhydroxid-lösungen eingesetzt.

Die einsetzende Reaktion ist exotherm und erfolgt entweder unter Rückfluß oder bei einem niedrig siedenden Alkylierungsmittel unter Druck und gegebenenfalls externer Kühlung, so daß die Reaktionstemperatur im Bereich von 50 bis 120 °C liegt.

Nach einer Reaktionszeit von insgesamt 2 bis 15 h wird der tert. Alkohol abdestilliert, der Rückstand mit Wasser aufgenommen und mit einem in Wasser unlöslichen Lösungsmittel wie z.B. Methylenchlorid, Ethylenchlorid, Ether, Trichlormethan, Trichlorethan oder Toluol extrahiert.

Die so erhaltenen Lösungen werden getrocknet. Danach wird das Lösungsmittel abdestilliert und aus dem Rückstand unter Vakuum das Dialkylisosorbid destilliert. Der rückgewonnene tert. Alkohol kann nach der Abtrennung von Wasser in einem weiteren Reaktionsansatz wieder eingesetzt werden.

In einer bevorzugten Ausführung des erfindungsgemäßen Verfahrens dient als Ausgangssubstanz nicht das teure 1,4;3,6-Dianhydrosorbitol, sondern das preiswerte Sorbitol, das in an sich bekannter Weise durch Abspalten von Wasser zu 1,4;3,6-Dianhydrosorbitol umgewandelt wird, welches dann ohne weitere Reinigung oder Isolierung erfindungsgemäß alkyliert wird.

### Beispiele

Beispiel 1: Dimethylisosorbid

292,3 g (2 Mol) 1,4;3,6-Dianhydrosorbitol und 800 ml tert. Butanol werden unter Rühren auf 55 - 65 °C erhitzt. Durch zwei verschiedene Tropftrichter werden in diesem Temperaturbereich gleichzeitig 572 g (7,14 Mol) 50 %ige Natronlauge und 631 g (5 Mol) Dimethylsulfat so zugetropft, daß das Reaktionsgemisch immer alkalisch ist. Man rührt 3 h bei ca. 60 °C nach und destilliert dann das tert. Butanol weitgehend ab. Zum Rückstand werden 2600 ml Wasser gegeben, die entstandene Lösung 3 x mit je 500 ml Methylenchlorid ausgerührt und die vereinigten organischen Auszüge mit Pottasche getrocknet. Man dampft zuerst das Lösungsmittel bei Normaldruck ab und destilliert dann das Produkt im Wasserstrahlvakuum.
Kp : 118 - 120 °C/20 mbar
Ausbeute: 324 g = 93 % der Theorie

**Beispiel 2: Diethylisosorbid**

292,3 g (2 Mol) 1,4;3,6-Dianhydrosorbitol und 800 ml tert. Butanol werden unter Rühren auf 70 - 80 °C erhitzt. Durch zwei verschiedene Tropftrichter werden bei dieser Temperatur 572 g (7,14 Mol) 50 %ige Natronlauge und 770 g (5 Mol) Diethylsulfat auf eine solche Weise zugetropft, daß das Reaktionsgemisch immer alkalisch ist. Zur Vervollkommnung der Reaktion rührt man noch 3 h bei der angegebenen Temperatur nach und destilliert dann das tert. Butanol weitgehend ab. Zum Rückstand werden 2600 ml Wasser gegeben und die entstehende Lösung 3 x mit je 500 ml Toluol ausgerührt und die vereinigten organischen Auszüge mit Pottasche getrocknet, das Lösungsmittel abgedampft und der Rückstand im Wasserstrahlvakuum destilliert.
Kp : 134 - 137 °C/20 mbar
Ausbeute: 368 g = 91 % der Theorie

**Beispiel 3: Dimethylisosorbid**

Analog Beispiel 1, jedoch mit folgenden Abwandlungen:
Tert. Amylalkohol anstelle tert. Butanol 800 g (7,14 Mol) 50 %ige Kalilauge anstelle Natronlauge Trichlormethan anstelle Methylenchlorid
Ausbeute: 318 g = 92 % der Theorie

**Beispiel 4: Dimethylisosorbid**

292,3 g (2 Mol) 1,4;3,4-Dianhydrosorbitol und 800 ml tert. Butanol werden unter Rühren auf 50 °C erhitzt. Durch zwei verschiedene Tropftrichter werden 710 g (5 Mol) Methyljodid und 572 g (7,14 Mol) 50 %ige Natronlauge so zugetropft, daß das Reaktionsgemisch immer alkalisch ist. Zur Vervollkommnung der Reaktion läßt man 12 h nachreagieren, wobei die Sumpftemperatur auf 80 °C gesteigert wird. Nun destilliert man den Alkohol ab und gibt zum Rückstand 1500 ml Wasser. Die entstehende Lösung wird 3 x mit je 500 ml Ether ausgerührt und die vereinigten organischen Auszüge mit Pottasche getrocknet, das Lösungsmittel abgedampft und der Rückstand im Wasserstrahlvakuum destilliert.
Kp : 118 - 126 °C/20 mbar
Ausbeute: 314 g = 91 % der Theorie

**Beispiel 5: Dimethylisosorbid**

Analog Beispiel 1, jedoch unter Verwendung von 930 g (5 Mol) 4-Toluolsulfonsäuremethylester als Alkylierungsmittel.
Ausbeute: 315 g = 91 % der Theorie

**Beispiel 6: Dimethylisosorbid**

1548 g (8,5 Mol) Sorbitol, 3000 ml Xylol und 20 ml konzentrierte Schwefelsäure werden unter Rühren zum Rückfluß erhitzt und ca. 306 ml Wasser ausgekreist. Man destilliert nun das Xylol ab (den letzten Rest im Vakuum) und nimmt den Rückstand in 3000 ml tert. Butanol auf, heizt auf 55 - 65 °C und tropft unter Rühren bei dieser Temperatur gleichzeitig 2432 g (30,4 Mol) 50 %ige Natronlauge und 2682 g (21,25 Mol) Dimethylsulfat so zu, daß das Gemisch immer alkalisch ist. Nach beendeter Zugabe rührt man 3 h bei 60 °C nach und destilliert dann den Alkohol ab bis zu einer Übergangstemperatur von 97 - 98 °C. Der Rückstand wird in 8500 ml Wasser aufgenommen und die wäßrige Lösung 3 x mit je 1000 ml Ethylenchlorid ausgerührt. Von den vereinigten organischen Phasen dampft man zunächst das Lösungsmittel ab, fraktioniert dann den Rückstand an einer Kolonne und sammelt die Fraktion mit dem Siedebereich von 118 - 120 °C/20 mbar.
Ausbeute: 1037 g = 70 % der Theorie

**Patentansprüche**

1. Verfahren zur Herstellung von 2,5-Dimethyl- bzw. 2,5-Diethyl-1,4;3,6-Dianhydrosorbitol in alkalischer wäßrig-organischer Lösung, **dadurch gekennzeichnet,** daß 1,4;3,6-Dianhydrosorbitol in tert. Butanol oder tert. Amylalkohol gelöst wird und bei einer Temperatur im Bereich von 50 bis 120 °C innerhalb von 2 bis 15 Stunden mit einem entsprechenden Alkylierungsmittel alkyliert wird, wobei Alkylierungsmittel und die wäßrige alkalische Lösung langsam getrennt so zugegeben werden, daß das Reaktionsgemisch immer alkalisch ist.

2. Verfahren nach Anspruch 1 **dadurch gekennzeichnet,** daß Sorbitol in an sich bekannter Weise durch Abspalten von Wasser zu 1,4;3,6-Dianhydrosorbitol umgesetzt wird, das dann ohne Isolierung oder Reinigung alkyliert wird.

**Claims**

1. A process for the preparation of 2,5-dimethyl- or 2,5-diethyl-1,4;3,6-dianhydrosorbitol in alkaline aqueous-organic solution, characterized in that 1,4;3,6-dianhydrosorbitol is dissolved in tert. butanol or tert. amyl alcohol and is alkylated with a suitable alkylating agent at a temperature in the range of from 50 to 120 °C within from 2 to 15 hours, alkylating agent and the aqueous alkaline solution being slowly added separately in such a way that the reaction mixture is always alkaline.

2. A process according to claim 1, characterized in that sorbitol is reacted In a manner known *per se* by splitting off water to form 1,4;3,6-dian-hydrosorbitol, which is then alkylated without isolation or purification.

**Revendications**

1. Procédé pour la préparation de 2,5-diméthyl- ou 2,5-diéthyl-1,4;3,6-dianhydrosorbitol en solution aqueuseorganique alcaline, caractérisé en ce que du 1,4;3,6-dianhy-drosorbitol est dissous dans du butanol tertiaire ou de l'alcool amylique tertiaire et est alkylé en l'espace de 2 à 15 heures à une température dans le domaine de 50 à 120°C avec un agent d'alkylation correspondant, l'agent d'alkylation et la solution alcaline aqueuse étant ajoutés lentement et séparément de telle manière que le mélange réactionnel soit toujours alcalin.

2. Procédé selon la revendication 1, caractérisé en ce que du sorbitol est transformé de façon connue en soi, par élimination d'eau, en 1,4;3,6-dianhydrosorbitol qui est ensuite alkylé sans isolement ou purification.